# EUROPEAN PATENT APPLICATION

(11) **EP 4 623 948 A1**
(43) Date of publication of application: **01.10.2025**
(21) Application number: 25164087.6
(22) Date of filing: 17.03.2025
(51) Int. Cl.: A61M 1/36

(54) **DIALYSIS APPARATUS**

(30) Priority: 26.03.2024 JP 2024049743
(71) Applicant: Shibuya Corporation, Kanazawa-shi, Ishikawa 920-8681 (JP)
(72) Inventor: FUJII, Masayuki, Ishikawa, 920-8681 (JP)
(74) Representative: Mewburn Ellis LLP

(57) **Abstract**

An object is to perform priming procedures efficiently. During a priming procedure, a state in which a vein passage 3A is connected to an upper position is maintained. While a dialysis fluid supply open/close valve V9 is closed, and a dialysis fluid retrieval open/close valve V10 is open, pressurizing device operates to cause a priming fluid to flow through a dialysis fluid retrieval passage 4B into a dialyzer 2. Thus, the priming fluid in a dialysis fluid flow path 12b is reversely filtered through a hollow fiber 12 (blood purification membrane), so that a blood flow path 12a is filled with the priming fluid and pressurized (b). Subsequently, after the dialysis fluid retrieval open/close valve V10 is closed, depressurizing device depressurizes a dialysis fluid circuit 4 to cause a pressure difference between a blood circuit 3 and the dialysis fluid circuit 4. When the dialysis fluid supply open/close valve V9 provided on a dialysis fluid supply passage 4A is opened, the priming fluid in the blood flow path 12a is, due to the pressure difference, filtered forward through the hollow fiber 12, and the priming fluid in the blood flow path 12a flows into the dialysis fluid flow path 12b, so that air bubbles B remaining in an upper part of the dialysis fluid flow path 12b are eliminated (c).

## Description

### BACKGROUND OF THE INVENTION

### Field of the Invention

The present invention relates to a dialysis apparatus and, more specifically, to a dialysis apparatus that makes it possible to efficiently perform a priming procedure by which a dialyzer is filled with a priming fluid.

### Description of the Related Art

Conventionally, a known dialysis apparatus used for performing dialysis treatment includes: a dialyzer, the interior of which is sectioned by a blood purification membrane into a blood flow path and a dialysis fluid flow path; a blood circuit connected to the blood flow path of the dialyzer; a dialysis fluid circuit connected to the dialysis fluid flow path of the dialyzer; and a fluid delivery pump that is provided on the dialysis fluid circuit and configured to deliver the dialysis fluid (Japanese Patent No. 7328531).

The abovementioned dialyzer has a tubular shape and is structured so that two end parts thereof are connected to an artery passage and to a vein passage serving as the blood circuit. When dialysis treatment is performed, the dialyzer is held in a state in which the part connected to the vein passage is facing upward.

Further, as for the abovementioned dialysis apparatus, before dialysis treatment is performed, it is necessary to attach the dialyzer to the dialysis apparatus and to perform a priming procedure by which the dialyzer, the artery passage, and the vein passage are filled with a priming fluid.

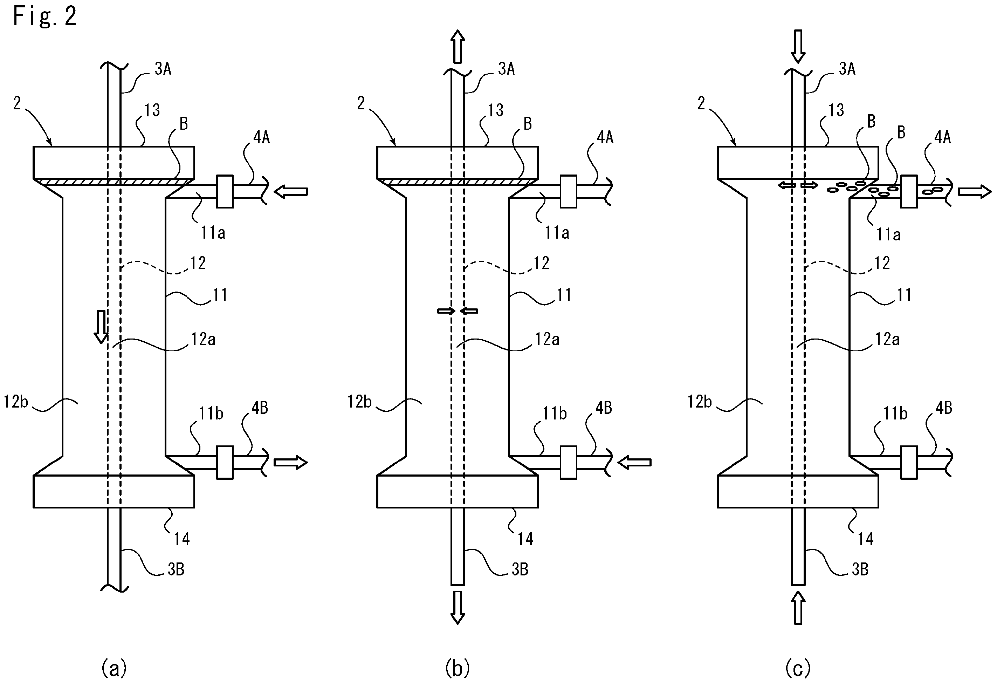

However, when the dialyzer is filled with the priming fluid during the abovementioned priming procedure, there is a possibility that air bubbles may remain inside the dialyzer. In particular, if the priming procedure is performed while the part connected to the vein passage is facing upward like during dialysis treatment, it is possible for air bubbles B to accumulate in an upper part of the dialyzer (see FIG. 2(a)).

To cope with this, it has been necessary to perform a procedure to eliminate the air bubbles from the dialyzer such as inverting the dialyzer upside down during the priming procedure. The procedure is cumbersome because monitoring and manual work of a worker are required.

In view of the problem described above, the present invention provides a dialysis apparatus that makes it possible to perform the priming procedure more efficiently, by eliminating the need for the monitoring and the manual work performed by the worker during the priming procedure.

### SUMMARY OF THE INVENTION

More specifically, the dialysis apparatus of the invention according to claim 1 is a dialysis apparatus including: a dialyzer, an interior of which is sectioned by a blood purification membrane into a blood flow path and a dialysis fluid flow path, a blood circuit including a vein passage and an artery passage connected to the blood flow path of the dialyzer, and a dialysis fluid circuit including a dialysis fluid supply passage and a dialysis fluid retrieval passage connected to the dialysis fluid flow path of the dialyzer, the dialysis apparatus being characterized in that
the dialysis apparatus includes a bypass passage allowing communication between the dialysis fluid supply passage and the dialysis fluid retrieval passage; a dialysis fluid supply open/close valve provided between a connection position at which the dialysis fluid supply passage is connected to the bypass passage and the dialyzer; a dialysis fluid discharge open/close valve provided between a connection position at which the dialysis fluid retrieval passage is connected to the bypass passage and the dialyzer; pressurizing device for pressurizing the dialysis fluid circuit; depressurizing device for depressurizing the dialysis fluid circuit; and controlling device for controlling these constituent elements,
during a priming procedure to fill the dialyzer with a priming fluid, the dialyzer is held in a state in which the vein passage is connected to an upper position, while the blood circuit is turned into a closed circuit,
the controlling device opens one of the dialysis fluid supply open/close valve and the dialysis fluid discharge open/close valve to cause the priming fluid to flow into the dialyzer through one of the dialysis fluid supply passage and the dialysis fluid retrieval passage, and by using the pressurizing device, causes the priming fluid to be reversely filtered through the blood purification membrane and to flow from the dialysis fluid flow path into the blood flow path,
when the blood circuit has been pressurized by the priming fluid, the controlling device closes the previously opened open/close valve to turn the dialysis fluid circuit into a closed circuit via the bypass passage, and causes the depressurizing device to depressurize the dialysis fluid circuit to cause a pressure difference between the blood circuit and the dialysis fluid circuit, and
the controlling device further opens the dialysis fluid supply open/close valve and causes, with the pressure difference between the blood circuit and the dialysis fluid circuit, the priming fluid in the blood flow path to be filtered forward through the blood purification membrane, so that the priming fluid flows into the dialysis fluid flow path and is discharged into the dialysis fluid supply passage together with air bubbles remaining in a upper part of the dialysis fluid flow path.

### Advantageous Effects of Invention

With the invention according to claim 1, at first, by using the pressurizing device, the priming fluid is caused to flow into the dialyzer through the one of the dialysis fluid supply passage and the dialysis fluid retrieval passage, so that the priming fluid is caused to be reversely filtered by the blood purification membrane and to flow into the blood flow path. However, because the dialyzer is held in the state in which the vein passage and the dialysis fluid retrieval passage are connected to upper positions, air bubbles would remain in an upper part.

To cope with this situation, the depressurizing device is caused to depressurize the dialysis fluid circuit to cause the pressure difference between the blood circuit and the dialysis fluid circuit. When the dialysis fluid supply open/close valve is opened in that state, the priming fluid in the blood flow path flows into the dialysis fluid flow path due to the pressure difference, and the air bubbles remaining in the upper part of the dialyzer are thus discharged.

With this configuration, it is possible to discharge the air bubbles from the dialyzer, without the need to invert the dialyzer. Because it is possible to eliminate the need for the monitoring and the work performed by the worker, it is possible to complete the priming work efficiently.

### BRIEF DESCRIPTION OF THE DRAWINGS

FIG. 1 is a circuit diagram of a dialysis apparatus according to an embodiment of the present disclosure;
FIG. 2 is a drawing for explaining a dialyzer;
FIG. 3 is a drawing for explaining steps in a priming procedure;
FIG. 4 is another drawing for explaining the steps in the priming procedure;
FIG. 5 is yet another drawing for explaining the steps in the priming procedure;
FIG. 6 is yet another drawing for explaining the steps in the priming procedure; and
FIG. 7 is a drawing for explaining steps in a priming procedure according to a second embodiment.

### DETAILED DESCRIPTION OF THE INVENTION

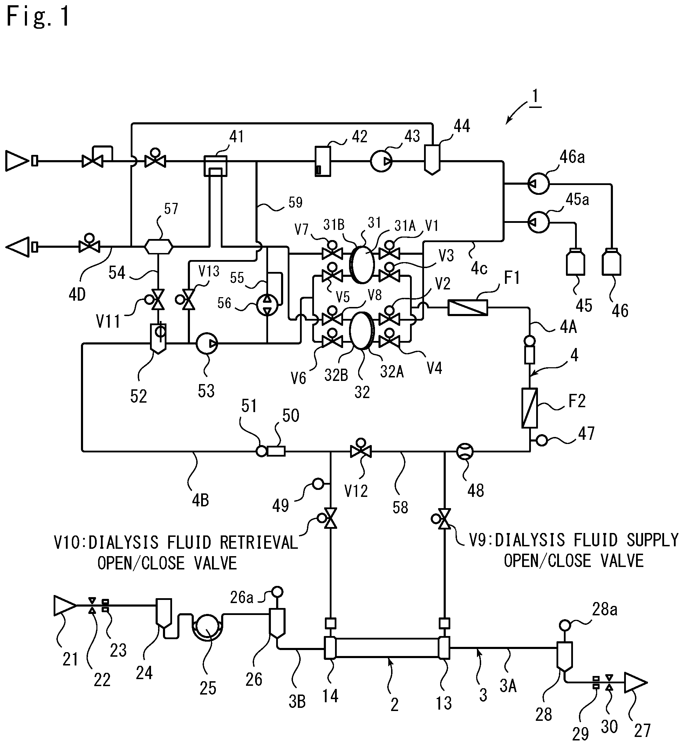

The present invention will be explained below by using the embodiments shown in the drawings. FIG. 1 shows a dialysis apparatus 1 including: a dialyzer 2 that performs hemodialysis; a blood circuit 3 that is connected to the dialyzer 2 and circulates blood; and a dialysis fluid circuit 4 that is connected to the dialyzer 2 and circulates a dialysis fluid. Further, the dialysis apparatus 1 is configured to be controlled by controlling device (not shown).

The dialysis apparatus 1 requires a priming procedure in which a new dialyzer 2 and a new blood circuit 3 are attached thereto before starting dialysis treatment so as to fill the dialyzer 2 and the blood circuit 3 with a priming fluid realized with saline, a dialysis fluid, or the like.

FIG. 2 is a drawing for explaining the dialyzer 2. FIG. 2(a) to 2(c) show states of the dialyzer 2 during the priming procedure.

The dialyzer 2 has a structure in which innumerable hollow fibers 12 serving as a blood purification membrane are accommodated inside a housing 11 having a tubular shape. The drawing schematically shows one hollow fiber 12, while the inside of the hollow fiber 12 is indicated as a blood flow path 12a communicating with the blood circuit 3, whereas the outside is indicated as a dialysis fluid flow path 12b communicating with the dialysis fluid circuit 4.

Provided at two end parts of the housing 11 are a vein-side head 13 connected to a vein passage 3A structuring the blood circuit 3 and an artery-side head 14 connected to an artery passage 3B.

In addition, on the lateral face of the housing 11, connection ports 11a and 11b each connected to the dialysis fluid circuit 4 are provided in positions adjacent to the vein-side head 13 and the artery-side head 14. Of these ports, the connection port 11a positioned on the side of the vein-side head 13 is connected to a dialysis fluid supply passage 4A structuring the dialysis fluid circuit 4, whereas the connection port 11b positioned on the side of the artery-side head 14 is connected to a dialysis fluid retrieval passage 4B.

Further, at the time of dialysis treatment and the priming procedure, the dialyzer 2 is configured to be attached in an upright state to the dialysis apparatus 1. More specifically, the dialyzer 2 is attached so that the vein-side head 13 is in an upper position, while the artery-side head 14 is in a lower position.

While the dialyzer 2 is attached in this manner, at the time of the dialysis treatment, the blood in the blood circuit 3 flows in from the lower position of the dialyzer 2 through the artery-side head 14 via the artery passage 3B, and flows through the blood flow path 12a within the hollow fiber 12 from the lower position toward the upper position, before being discharged into the vein passage 3A through the vein-side head 13 provided in the upper position.

Meanwhile, as for the dialysis fluid in the dialysis fluid circuit 4, fresh dialysis fluid flows in from the dialysis fluid supply passage 4A through the connection port 11a provided in the upper position of the dialyzer 2, and flows through the dialysis fluid flow path 12b provided outside the hollow fiber 12, from the upper position toward the lower position, before being discharged into the dialysis fluid retrieval passage 4B through the connection port 11b provided in the lower position of the dialyzer 2.

Further, while the blood is flowing through the blood flow path 12a and the dialysis fluid is flowing through the dialysis fluid flow path 12b, hemodialysis is performed in the hollow fiber 12 serving as the blood purification membrane.

The blood circuit 3 includes the artery passage 3B that is connected to a blood vessel of a patient and supplies blood toward the dialyzer 2 and the vein passage 3A that returns blood from the dialyzer 2 to a blood vessel of the patient.

One end of the artery passage 3B is connected to the artery-side head 14 of the dialyzer 2, while a puncture needle 21 punctured into a blood vessel of the patient is provided at the other end. The artery passage 3B is provided with a clamp 22, an air bubble sensor 23, a drip chamber 24, a blood pump 25, a drip chamber 26, and a pressure sensor 26a provided on the drip chamber 26.

One end of the vein passage 3A is connected to the vein-side head 13 of the dialyzer 2, while a puncture needle 27 punctured into a blood vessel of the patient is provided at the other end. The vein passage 3A is provided with a drip chamber 28, a pressure sensor 28a provided on the drip chamber 28, an air bubble sensor 29, and a clamp 30.

Further, at the time of priming the dialyzer 2 with the dialysis fluid, a closed circuit that is not externally opened to the air is formed by connecting a tip end part of the artery passage 3B and a tip end part of the vein passage 3A to each other.

The dialysis fluid circuit 4 includes a first dialysis fluid chamber 31 and a second dialysis fluid chamber 32 having an identical shape and storing the dialysis fluid therein. Formed inside the first and the second dialysis fluid chambers 31 and 32 are supply compartments 31A and 32A storing fresh dialysis fluid therein and retrieval compartments 31B and 32B storing used dialysis fluid therein.

To each of the supply compartments 31A and 32A, a fluid feed passage 4C and the dialysis fluid supply passage 4A that are branched are connected. The branched passages of the fluid feed passage 4C are provided with fluid feed valves V1 and V2. The branched passages of the dialysis fluid supply passage 4A are provided with supply valves V3 and V4.

Meanwhile, to each of the retrieval compartments 31B and 32B, the dialysis fluid retrieval passage 4B and a fluid discharge passage 4D that are each branched are connected. The branched passages of the dialysis fluid retrieval passage 4B are provided with retrieval valves V5 and V6. The branched passages of the fluid discharge passage 4D are provided with fluid discharge valves V7 and V8.

The fluid feed passage 4C is provided with purified water supply device (not shown) for supplying purified water to the upstream side thereof and is also provided with a heat exchanger 41, a heater 42, an exhaust pump 43, and a deaeration tank 44, while a fluid A supply source 45 and a fluid B supply source 46 for undiluted fluids of the dialysis fluid are connected en route. Further, undiluted fluid A and undiluted fluid B are delivered from the fluid A supply source 45 and the fluid B supply source 46, by a fluid A pump 45a and a fluid B pump 46a, respectively.

The dialysis fluid supply passage 4A is provided with a first dialysis fluid filter F1 and a second dialysis fluid filter F2 each realized with an endotoxin removal filter that cleans the dialysis fluid, a fluid pressure sensor 47, and a flowmeter 48. Provided between the flowmeter 48 and the dialyzer 2 is a ninth open/close valve V9 serving as a dialysis fluid supply open/close valve.

The dialysis fluid retrieval passage 4B is provided with a tenth open/close valve V10 serving as a dialysis fluid discharge open/close valve, a fluid pressure sensor 49, a concentration sensor 50, a temperature sensor 51, a deaeration tank 52, and a fluid delivery pump 53.

The deaeration tank 52 structures depressurizing device in a first embodiment. A deaeration passage 54 is provided between the deaeration tank 52 and the fluid discharge passage 4D. The deaeration passage 54 is provided with a deaeration valve V11 serving as a deaeration passage open/close valve.

By eliminating air bubbles from the fluid flowing through the dialysis fluid retrieval passage 4B and opening the deaeration valve V11 of the deaeration passage 54, the deaeration tank 52 is capable of discharging only the air bubbles (air) contained in the deaeration tank 52 into the fluid discharge passage 4D via the deaeration passage 54.

A water removal passage 55 communicating with the fluid discharge passage 4D is connected to a position adjacent to the downstream side of the fluid delivery pump 53. The water removal passage 55 is provided with a water removal pump 56.

The fluid discharge passage 4D is connected to a fluid discharge tank (not shown), while a buffer tank 57 connected to the deaeration passage 54 is provided en route.

Further, the dialysis apparatus 1 according to the present embodiment is provided with a first bypass passage 58 between the dialysis fluid supply passage 4A and the dialysis fluid retrieval passage 4B. The first bypass passage 58 is provided with a twelfth open/close valve V12.

One end of the first bypass passage 58 is situated on the dialysis fluid supply passage 4A, while being positioned on the upstream side of the ninth open/close valve V9 and positioned between the flowmeter 48 and the ninth open/close valve V9. The other end is situated on the dialysis fluid retrieval passage 4B, while being positioned on the downstream side relative to the tenth open/close valve V10 and positioned between the fluid pressure sensor 49 and the concentration sensor 50.

Further, a second bypass passage 59 structuring pressurizing device in the first embodiment is provided between the fluid feed passage 4C and the dialysis fluid retrieval passage 4B. The second bypass passage 59 is provided with a thirteenth open/close valve V13.

One end of the second bypass passage 59 is situated on the dialysis fluid retrieval passage 4B, while being positioned on the upstream side relative to the fluid delivery pump 53 and positioned between the deaeration tank 52 and the fluid delivery pump 53. The other end is situated on the dialysis fluid retrieval passage 4B, while being positioned between the heat exchanger 41 and the heater 42.

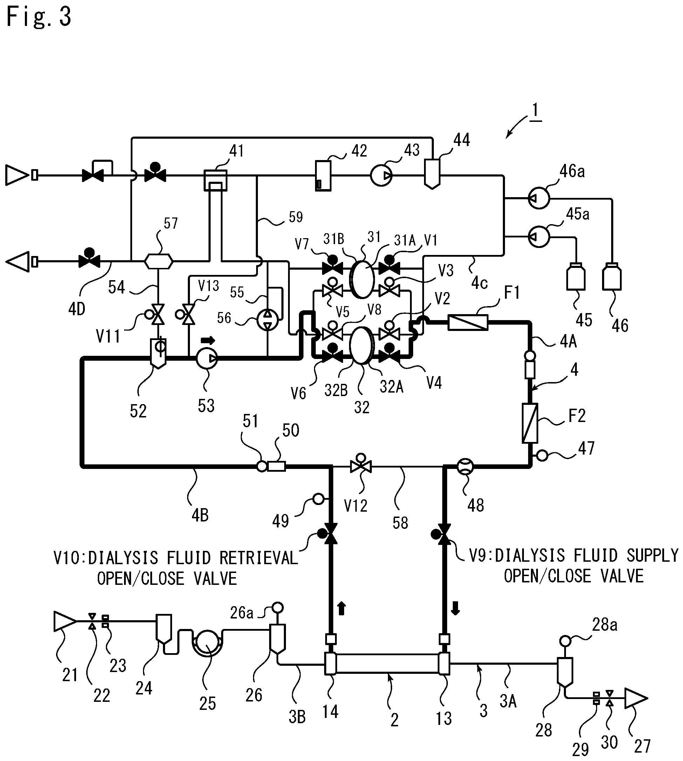

With regard to the dialysis apparatus 1 having the structure described above, a flow of the dialysis fluid while hemodialysis treatment is performed will be explained with reference to FIG. 3. Flows of the water and the dialysis fluid through the fluid feed passage 4C and the fluid discharge passage 4D are omitted from the drawing. Further, in the following explanations, the open/close valves are indicated with solid black while being open and are indicated with solid white while being closed.

To the supply compartment 31A in the first dialysis fluid chamber 31, water, the undiluted fluid A, and the undiluted fluid B are supplied from water supply device, the fluid A supply source 45, and the fluid B supply source 46 via the fluid feed passage 4C and are mixed therein, so that fresh dialysis fluid is prepared.

When the water and the undiluted fluids have flowed into the supply compartment 31A, the volume of the supply compartment 31A increases while a diaphragm is changing the shape thereof. In conjunction therewith, the volume of the retrieval compartment 31B decreases, and used dialysis fluid is discharged from the retrieval compartment 31B via the fluid discharge passage 4D.

In contrast, to the retrieval compartment 32B in the second dialysis fluid chamber 32, the used dialysis fluid past the dialyzer 2 is supplied via the dialysis fluid retrieval passage 4B. As a result, when the volume of the retrieval compartment 32B has increased, the volume of the supply compartment 32A decreases in conjunction therewith, so that fresh dialysis fluid is delivered from the supply compartment 32A to the dialyzer 2 via the dialysis fluid supply passage 4A.

Further, when the volume of the retrieval compartment 31B in the first dialysis fluid chamber 31 and the supply compartment 32A in the second dialysis fluid chamber 32 become zero, the open/close states of the valves are switched between the fluid feed valves V1 and V2, the fluid discharge valves V7 and V8, the supply valves V3 and V4, and the retrieval valves V5 and V6 provided on the first and the second dialysis fluid chambers 31 and 32.

As a result, in the first dialysis fluid chamber 31, fresh dialysis fluid is supplied from the supply compartment 31A to the dialyzer 2, whereas used dialysis fluid is retrieved into the retrieval compartment 31B. In contrast, in the second dialysis fluid chamber 32, fresh dialysis fluid is supplied to the supply compartment 32A, whereas used dialysis fluid is discharged from the retrieval compartment 32B.

After that, by repeatedly performing the above operations alternately, it is possible to continuously supply fresh dialysis fluid and to retrieve used dialysis fluid to and from the dialyzer 2, by employing the dialysis fluid circuit 4. Thus, hemodialysis is performed by the dialyzer 2 with the blood circulated in the blood circuit 3.

Further, when a water removal operation is performed so as to remove excess water from blood of the patient during dialysis treatment, the water removal pump 56 provided on the water removal passage 55 is brought into operation, so as to discharge a part of the used dialysis fluid flowing through the dialysis fluid retrieval passage 4B into the fluid discharge passage 4D via the water removal passage 55 so that, due to a pressure difference thereby caused inside the dialyzer 2, the excess water is removed from the blood.

Next, a priming procedure using the dialysis apparatus 1 according to the present embodiment will be explained, with reference to FIGS. 3 to 6. In the present example, the procedure performed at the time of filling the dialysis fluid flow path 12b and the blood flow path 12a of the dialyzer 2 with a dialysis fluid will be explained. Details of a procedure to fill the vein passage 3A and the artery passage 3B with the dialysis fluid will be omitted.

To begin with, the dialyzer 2 and the blood circuit 3 that are empty are connected to the dialysis apparatus 1. In this situation, the dialyzer 2 is attached while the vein-side head 13 is facing upward like during dialysis treatment. In addition, the closed circuit that is not externally opened to the air is formed by connecting together an end part of the artery passage 3B and an end part of the vein passage 3A in the blood circuit 3.

In the state described above, when an operation to start the priming procedure is performed on the controlling device, a procedure to fill the dialysis fluid flow path 12b in the dialyzer 2 with the dialysis fluid is performed at first.

More specifically, like during dialysis treatment, the fluid delivery pump 53 is brought into operation to cause the dialysis fluid to be circulated in the dialysis fluid circuit 4. As a result, fresh dialysis fluid flows into the dialyzer 2 through the dialysis fluid supply passage 4A, so that the dialysis fluid flow path 12b positioned outside the hollow fiber 12 in the dialyzer 2 is filled with the dialysis fluid.

In this situation, in the present embodiment, the priming is performed as shown in FIG. 2 while the dialyzer 2 is maintained in the upright state in which the vein-side head 13 is in the upper position. Accordingly, in the operation shown in FIG. 3, the dialysis fluid flows into the dialysis fluid flow path 12b of the dialyzer 2, through the dialysis fluid supply passage 4A connected to the connection port 11a positioned in the upper part of the housing 11 as shown in FIG. 2(a).

However, because the dialysis fluid that has flowed in flows downward from the connection port 11a, and discharged through the connection port 11b, can't be able to reach as far as the position above the connection port 11a. As a result, it is possible for air bubbles B to remain in the upper part of the dialysis fluid flow path.

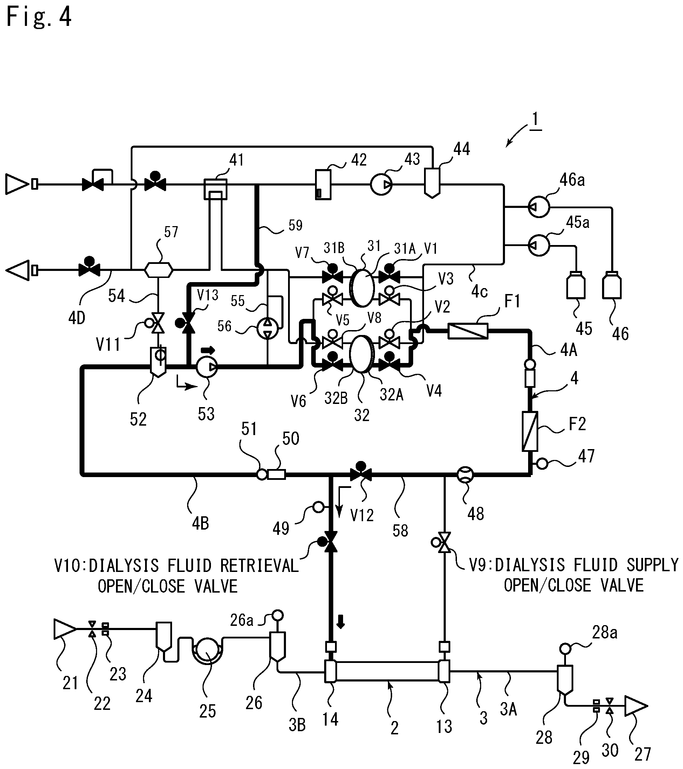

FIG. 4 shows an operation in which, by using the pressurizing device, the dialysis fluid is caused to be reversely filtered through the hollow fiber 12 in the dialyzer 2, so as to flow from the dialysis fluid flow path 12b into the blood flow path 12a.

In the present embodiment, the abovementioned pressurizing device comprises the fluid delivery pump 53 provided on the dialysis fluid retrieval passage, the second bypass passage 59, and the thirteenth open/close valve V13 described above.

From the state shown in FIG. 3, the controlling device closes the ninth open/close valve V9 provided on the dialysis fluid supply passage 4A and opens the twelfth open/close valve V12 provided on the first bypass passage 58 and the thirteenth open/close valve V13 provided on the second bypass passage 59.

As a result, for example, the fresh dialysis fluid discharged from the supply compartment 32A in the second dialysis fluid chamber 32 flows through the dialysis fluid supply passage 4A, before flowing into the first bypass passage 58.

Meanwhile, because the controlling device has the fluid delivery pump 53 provided on the dialysis fluid retrieval passage 4B in operation, while the second bypass passage 59 is connected to the upstream side of the fluid delivery pump 53, the fluid delivery pump 53 supplies the water supplied via the second bypass passage 59 to the retrieval compartment 32B in the second dialysis fluid chamber 32.

As a result, in the part positioned on the upstream side relative to the connection position at which the dialysis fluid retrieval passage 4B is connected to the second bypass passage 59, the flow of the dialysis fluid toward the retrieval compartment 32B becomes stagnant. Thus, the dialysis fluid flowing through the first bypass passage 58 flows backward toward the dialyzer 2, from the connection part in which the dialysis fluid retrieval passage 4B is connected to the first bypass passage 58.

The dialysis fluid that has flowed backward through the dialysis fluid retrieval passage 4B in this manner flows past the tenth open/close valve V10 provided on the dialysis fluid retrieval passage 4B and flows into the dialysis fluid flow path 12b of the dialyzer 2. On the inside of the dialyzer 2, the dialysis fluid flow path 12b is pressurized and thus has positive pressure relative to the blood flow path 12a, so that there is a pressure difference between the dialysis fluid flow path 12b and the blood flow path 12a.

As a result, reverse filtering occurs in the hollow fiber 12, so that the dialysis fluid flows from the dialysis fluid flow path 12b into the blood flow path 12a. As a result of keeping this state for a prescribed period of time, the blood flow path 12a will be filled with the dialysis fluid.

In this situation, because the blood circuit 3 forms a closed circuit, while the blood pump 25 has been stopped, although the dialysis fluid having flowed into the blood flow path 12a flows into parts of the vein passage 3A and the artery passage 3B, flowing beyond those is inhibited, and the pressure in the blood circuit 3 also increases due to the inflow of the dialysis fluid.

FIG. 2(b) shows a state of the dialyzer 2 during the operation shown in FIG. 4, where the priming is performed while the upright state is maintained so that the vein-side head 13 is in the upper position.

After flowing in through the connection port 11b on the side of the artery-side head 14 provided in the lower position, the dialysis fluid is reversely filtered and flows from the dialysis fluid flow path 12b into the blood flow path 12a. However, because the hollow fiber 12 does not pass air bubbles, air bubbles B remain in the position above the connection port 11a that is between the connection port 11a and the vein-side head 13.

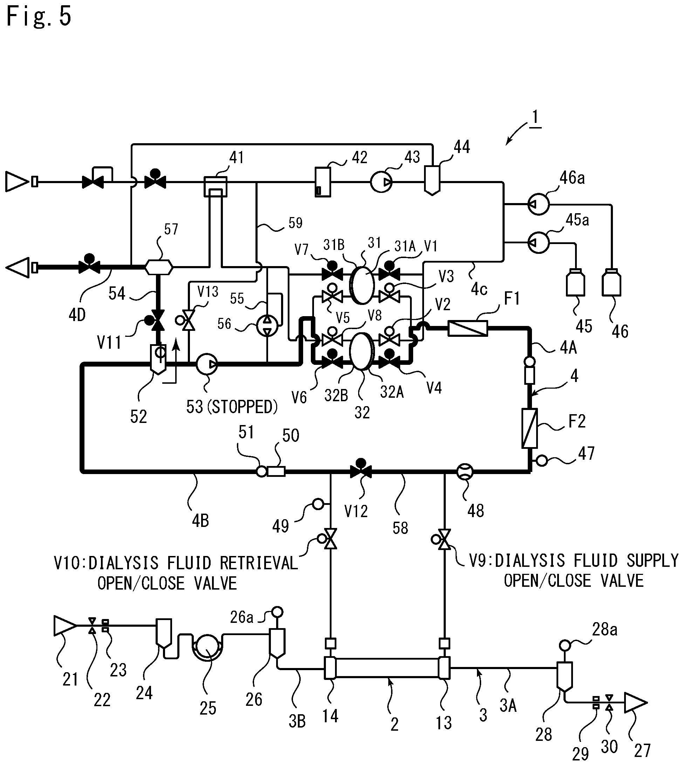

FIG. 5 shows an operation in which the depressurizing device depressurizes the dialysis fluid circuit 4 to cause a pressure difference between the blood circuit 3 and the dialysis fluid circuit 4.

In the present embodiment, the depressurizing device is structured with the deaeration tank 52 provided on the dialysis fluid retrieval passage 4B; and the deaeration passage 54 and the deaeration valve V11 connected to the deaeration tank 52.

During the operation shown in FIG. 4, the controlling device is measuring the pressure in the blood circuit 3 by using the pressure sensors 26a and 28a provided on the drip chambers 26 and 28 respectively positioned on the artery passage 3B and the vein passage 3A in the blood circuit 3. When the pressure in the blood circuit 3 reaches a prescribed level, the controlling device closes the tenth open/close valve V10 provided on the dialysis fluid retrieval passage 4B and closes the thirteenth open/close valve V13 provided on the second bypass passage 59.

As a result, the dialysis fluid circuit 4 forms a closed circuit that is not externally open to the air and includes the dialysis fluid supply passage 4A, the first bypass passage 58, and the dialysis fluid retrieval passage 4B.

In that state, the controlling device opens the deaeration valve V11 serving as the depressurizing device and stops the fluid delivery pump 53 on the dialysis fluid retrieval passage 4B.

As a result, the air discharged through the dialysis fluid flow path 12b of the dialyzer 2 in the operations shown in FIG. 3 and FIG. 4 flows through the dialysis fluid retrieval passage 4B together with the dialysis fluid and is retrieved into the deaeration tank 52, so that the air bubbles are discharged through the fluid discharge passage 4D via the deaeration passage 54.

Because the dialysis fluid circuit 4 has the closed circuit formed by the dialysis fluid supply passage 4A, the first bypass passage 58, and the dialysis fluid retrieval passage 4B, the volume of the closed circuit decreases due to the discharging of the air through the deaeration passage 54, and the dialysis fluid circuit 4 is thus depressurized.

Meanwhile, the dialyzer 2 and the blood circuit 3 also has the closed circuits formed, and as a result of the operation shown in FIG. 4, those closed circuits each have positive pressure. In addition, because the tenth open/close valve V10 on the dialysis fluid retrieval passage 4B was closed in the operation shown in FIG. 5, the positive pressure is maintained.

In other words, as a result of the operation in FIG. 5, the pressure of the dialysis fluid circulated in the dialysis fluid circuit 4 becomes lower than the pressure of the dialysis fluid circulated through the blood circuit 3, so that there is a pressure difference between the two.

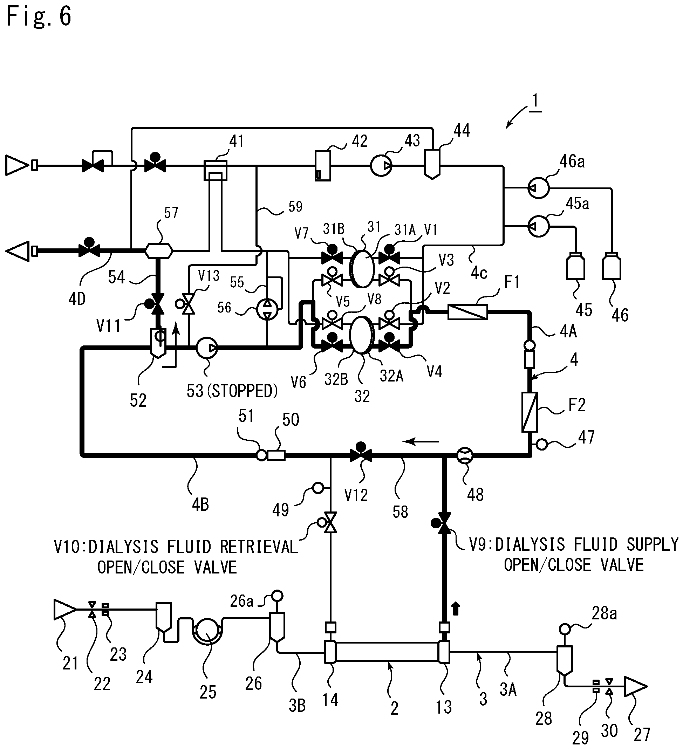

FIG. 6 shows an operation in which, with the pressure difference between the blood circuit 3 and the dialysis fluid circuit 4, the dialysis fluid in the blood flow path 12a is filtered forward through the hollow fiber 12, so as to use the dialysis fluid that has flowed into the dialysis fluid flow path 12b for discharging the air bubbles remaining in the upper part into the dialysis fluid supply passage 4A.

The controlling device is measuring the pressure in the dialysis fluid circuit 4 by using the fluid pressure sensor 47 provided on the dialysis fluid supply passage 4A. When the measured pressure has dropped to reach a prescribed level, the controlling device opens the ninth open/close valve V9 provided on the dialysis fluid supply passage 4A. Alternatively, the opening of the ninth open/close valve V9 does not necessarily need to be triggered by the pressure measured by the fluid pressure sensor 47 and may be triggered when a prescribed period of time has elapsed.

When the ninth open/close valve V9 is opened, the blood circuit 3 and the dialysis fluid circuit 4 communicate with each other via the dialysis fluid supply passage 4A, and due to a pressure difference between the two, the dialysis fluid in the dialyzer 2, which had the positive pressure, is discharged toward the dialysis fluid supply passage 4A.

FIG. 2(c) shows a state of the dialyzer 2 during the operation shown in FIG. 6, which is the upright state so that the vein-side head 13 is in the upper position. As a result of the operation shown in FIG. 6, the dialysis fluid is filtered forward from the blood flow path 12a into the dialysis fluid flow path 12b so that, in the upper part of the dialyzer 2 also, an inflow of the dialysis fluid into the dialysis fluid flow path 12b occurs. The air bubbles B remaining in the upper part are replaced by the dialysis fluid, are pushed out of the upper part, and are discharged into the dialysis fluid supply passage 4A through the connection port 11a provided in the upper position. After that, the air bubbles B are discharged from the dialysis fluid supply passage 4A into the dialysis fluid retrieval passage 4B via the first bypass passage 58.

This procedure may be performed until a prescribed period of time has elapsed; however, detection by a sensor or the like may be used as a trigger. After that, the controlling device closes the ninth open/close valve V9 and, as a result of bringing the fluid delivery pump 53 into operation, the air bubbles B are retrieved into the deaeration tank 52.

As described above, because the air bubbles are eliminated from the dialysis fluid flow path 12b of the dialyzer 2 by the operation shown in FIG. 6, the blood flow path 12a and the dialysis fluid flow path 12b are completely filled with the dialysis fluid, and the priming of the dialyzer 2 is thus completed.

After that, it is suggested that the blood circuit 3 be filled with a priming fluid by implementing a method that is hitherto known publicly. For example, the blood circuit 3 may be connected to an infusion bag storing the priming fluid therein such as a dialysis fluid or saline, and by bringing the blood pump 25 into operation in that state, it is possible to fill the blood circuit 3 with the priming fluid.

As explained above, when the dialysis apparatus 1 according to the present embodiment is used, when the priming procedure is performed on the dialyzer 2 and the blood circuit 3, it is possible to eliminate the air bubbles B even when the dialyzer 2 is maintained in the upright state where the vein-side head 13 is in the upper position. It is therefore possible to eliminate the procedure of inverting the dialyzer 2 upside down and to thus reduce burdens on the worker.

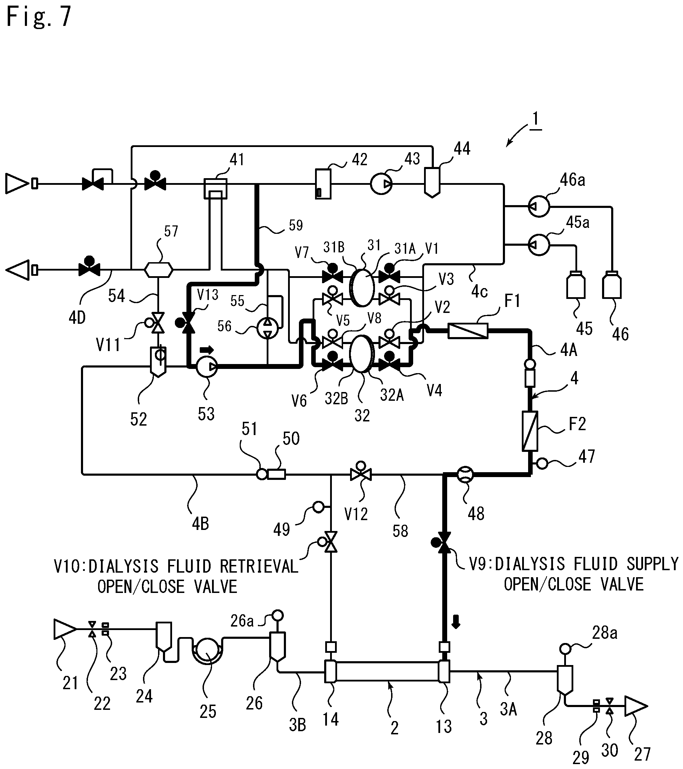

FIG. 7 shows a priming procedure using the dialysis apparatus 1 according to a second embodiment and corresponds to the procedure shown in FIG. 4 of the first embodiment.

In the first embodiment, at the time of reversely filtering the dialysis fluid from the dialysis fluid flow path 12b into the blood flow path 12a in the dialyzer 2, the tenth open/close valve V10 provided on the dialysis fluid retrieval passage 4B is opened to cause the dialysis fluid to flow in through the connection port 11b provided in the lower position of the dialyzer 2.

In contrast, in the second embodiment, a configuration is used in which the dialysis fluid is caused to flow in through the connection port 11a provided in the upper position and connected to the dialysis fluid supply passage 4A.

More specifically, from the state in which the dialysis fluid flow path 12b of the dialyzer 2 is filled with the dialysis fluid in the state in FIG. 3, the controlling device closes the tenth open/close valve V10 provided on the dialysis fluid retrieval passage 4B and the twelfth open/close valve V12 provided the first bypass passage 58, as shown in FIG. 7.

As a result, for example, fresh dialysis fluid discharged from the supply compartment 32A in the second dialysis fluid chamber 32 flows through the dialysis fluid supply passage 4A and flows into the dialysis fluid flow path 12b of the dialyzer 2. On such occasion, the twelfth open/close valve V12 of the first bypass passage 58 may be kept open without being closed.

In this situation, because the dialyzer 2 is in the upright state so that the vein-side head 13 is in the upper position, the dialysis fluid flows in through the connection port 11a provided in the upper position, and subsequently, is reversely filtered and flows from the dialysis fluid flow path 12b into the blood flow path 12a; however, because the hollow fiber 12 does not pass air bubbles, the air bubbles B remain, after all, in the position above the connection port 11a that is between the connection port 11a and the vein-side head 13.

Further, after the operation in FIG. 7, by performing the operations shown in FIGS. 5 and 6 similarly to the first embodiment, it is possible to eliminate the air bubbles from the dialyzer 2 without changing the posture of the dialyzer 2.

In the first and the second embodiments described above, the deaeration tank 52 and the deaeration valve V11 provided on the deaeration passage 54 are used as the depressurizing device for depressurizing the dialysis fluid circuit 4, so as to depressurize the dialysis fluid circuit 4 by discharging the air from the deaeration tank 52 during the operation shown in FIG. 5.

Alternatively, it is also acceptable to use the water removal passage 55 and the water removal pump 56 as the depressurizing device. In other words, in the operation shown in FIG. 5, instead of discharging the air by opening the deaeration valve V11, the water removal pump 56 may be brought into operation to cause the dialysis fluid in the dialysis fluid retrieval passage 4B to flow into the fluid discharge passage 4D.

With this configuration, the closed circuit formed by the dialysis fluid supply passage 4A, the first bypass passage 58, and the dialysis fluid retrieval passage 4B is depressurized, and it is therefore possible to cause a pressure difference between the blood circuit 3 and the dialysis fluid circuit 4.

Further, in the first and the second embodiments described above, the fluid delivery pump 53 provided on the dialysis fluid retrieval passage 4B and the second bypass passage 59 are used as the pressurizing device for pressurizing the dialysis fluid circuit 4.

Alternatively, it is also acceptable to use the water removal pump 56 provided on the water removal passage 55 as the pressurizing device, while omitting the second bypass passage 59.

In that situation, in the operations shown in FIG. 4 and FIG. 7, the fluid delivery pump 53 is stopped, while the water removal pump 56 is operated toward the opposite side as compared to during the water removal operation. With this configuration, the dialysis fluid in the fluid discharge passage 4D flows into the dialysis fluid retrieval passage 4B and subsequently flows into the retrieval compartment 32B in the second dialysis fluid chamber 32.

As a result, from the supply compartment 32A in the second dialysis fluid chamber 32, the dialysis fluid is discharged in an amount corresponding to a discharge volume of the water removal pump 56. Thus, similarly to the first and the second embodiments, the dialysis fluid in the dialysis fluid supply passage 3A may flow backward through the first bypass passage 58 and a part of the dialysis fluid retrieval passage 4B before flowing into the dialyzer 2 or may flow through the dialysis fluid supply passage 4A before flowing into the dialyzer 2 and is thus reversely filtered from the dialysis fluid flow path 12b into the blood flow path 12a.

On such occasion, it is desirable to configure the discharge volume of the water removal pump 56 to be equal to or larger than the volume of the passage between the connection position at which the dialysis fluid retrieval passage 4B is connected to the first bypass passage 58 and the connection port 11b or equal to or larger than the volume of the passage between the connection position at which the dialysis fluid supply passage 4A is connected to the first bypass passage 58 and the connection port 11a.

Further, the above embodiments were explained regarding a so-called personal dialysis apparatus configured to prepare the dialysis fluid in the first and the second dialysis fluid chambers 31 and 32; however, the present disclosure is also applicable to a dialysis monitoring apparatus configured to use a dialysis fluid prepared in advance.

Furthermore, the above embodiments were explained regarding the dialysis apparatus including no fluid replenish passage allowing communication between the dialysis fluid supply passage 4A and either the vein passage 3A or the artery passage 3B; however, the present disclosure is also applicable to an apparatus including such a fluid replenish passage.

### Reference Signs List

1: dialysis apparatus 2: dialyzer
3: blood circuit 3A: vein passage
3B: artery passage 4: dialysis fluid circuit
4A: dialysis fluid supply passage
4B: dialysis fluid retrieval passage
11: housing
12: hollow fiber (blood purification membrane)
13: vein-side head 14: artery-side head
53: fluid delivery pump
58: first bypass passage
52: deaeration tank (depressurizing device)
54: deaeration passage (depressurizing device)
V9: ninth open/close valve (dialysis fluid supply open/close valve)
V10: tenth open/close valve (dialysis fluid discharge open/close valve)
B: air bubble

## Claims

1. A dialysis apparatus including: a dialyzer, an interior of which is sectioned by a blood purification membrane into a blood flow path and a dialysis fluid flow path, a blood circuit including a vein passage and an artery passage connected to the blood flow path of the dialyzer, and a dialysis fluid circuit including a dialysis fluid supply passage and a dialysis fluid retrieval passage connected to the dialysis fluid flow path of the dialyzer, the dialysis apparatus being **characterized in that**
the dialysis apparatus comprises a bypass passage allowing communication between the dialysis fluid supply passage and the dialysis fluid retrieval passage; a dialysis fluid supply open/close valve provided between a connection position at which the dialysis fluid supply passage is connected to the bypass passage and the dialyzer; a dialysis fluid discharge open/close valve provided between a connection position at which the dialysis fluid retrieval passage is connected to the bypass passage and the dialyzer; pressurizing device for pressurizing the dialysis fluid circuit; depressurizing device for depressurizing the dialysis fluid circuit; and controlling device for controlling these constituent elements,
during a priming procedure to fill the dialyzer with a priming fluid, the dialyzer is held in a state in which the vein passage is connected to an upper position, while the blood circuit is turned into a closed circuit,
the controlling device opens one of the dialysis fluid supply open/close valve and the dialysis fluid discharge open/close valve to cause the priming fluid to flow into the dialyzer through one of the dialysis fluid supply passage and the dialysis fluid retrieval passage, and by using the pressurizing device, causes the priming fluid to be reversely filtered through the blood purification membrane and to flow from the dialysis fluid flow path into the blood flow path,
when the blood circuit has been pressurized by the priming fluid, the controlling device closes the previously opened open/close valve to turn the dialysis fluid circuit into a closed circuit via the bypass passage, and causes the depressurizing device to depressurize the dialysis fluid circuit to cause a pressure difference between the blood circuit and the dialysis fluid circuit, and
the controlling device further opens the dialysis fluid supply open/close valve and causes, with the pressure difference between the blood circuit and the dialysis fluid circuit, the priming fluid in the blood flow path to be filtered forward through the blood purification membrane, so that the priming fluid flows into the dialysis fluid flow path and is discharged into the dialysis fluid supply passage together with air bubbles remaining in a upper part of the dialysis fluid flow path.

2. The dialysis apparatus according to claim 1, **characterized in that**
the depressurizing device includes a deaeration tank that is for eliminating air and is provided on the dialysis fluid retrieval passage, a deaeration passage connected to the deaeration tank, and a deaeration passage open/close valve for opening and closing the deaeration passage,
when causing the depressurizing device to depressurize the dialysis fluid circuit, the controlling device opens the deaeration passage open/close valve, and
the dialysis fluid circuit is depressurized as a result of the air inside the deaeration tank being discharged through the deaeration passage.

3. The dialysis apparatus according to claim 1, **characterized in that**
the depressurizing device includes a water removal passage connected to the dialysis fluid retrieval passage and a water removal pump provided on the water removal passage,
when causing the depressurizing device to depressurize the dialysis fluid circuit, the controlling device brings the water removal pump into operation, and
the dialysis fluid circuit is depressurized as a result of discharging, through the water removal passage, the priming fluid flowing through the dialysis fluid retrieval passage.

4. The dialysis apparatus according to claim 1, **characterized in that**
the dialysis fluid circuit includes a dialysis fluid chamber sectioned, by a diaphragm, into a supply compartment connected to the dialysis fluid supply passage and a retrieval compartment connected to the dialysis fluid retrieval passage,
the pressurizing device includes a fluid delivery pump provided on the dialysis fluid retrieval passage and a second bypass passage that is connected to an upstream side relative to the fluid delivery pump and causes the priming fluid to flow therein,
when causing the priming fluid to be reversely filtered through the blood purification membrane by using the pressurizing device, the controlling device brings the fluid delivery pump into operation, so that the priming fluid from the second bypass passage is supplied to the retrieval compartment,
in conjunction therewith, the priming fluid discharged from the supply compartment into the dialysis fluid supply passage flows into the dialyzer through one of the dialysis fluid supply passage and the dialysis fluid retrieval passage, and is thereby reversely filtered through the blood purification membrane.

5. The dialysis apparatus according to claim 1, **characterized in that**
the pressurizing device includes a water removal passage connected to the dialysis fluid retrieval passage and a water removal pump provided on the water removal passage, and
when causing the priming fluid to be reversely filtered through the blood purification membrane by using the pressurizing device, the controlling device brings the water removal pump into operation to cause the priming fluid to flow from the water removal passage into the dialysis fluid retrieval passage and to thereby cause the priming fluid to be reversely filtered through the blood purification membrane.
